# EUROPEAN PATENT APPLICATION

(11) **EP 2 051 073 A1**
(43) Date of publication of application: **22.04.2009**
(21) Application number: 08166915.2
(22) Date of filing: 17.10.2008
(51) Int. Cl.: G01N 33/53, G01N 33/68

(54) **Methods and kits for predicting risk for preterm labor**

(30) Priority: 18.10.2007 US 874717
(71) Applicant: Cervimark II, LLC, St. Louis, MO 63117 (US)
(72) Inventor: Kokenyesi, Robert, Kirkwood, MO 63122 (US); Rai, Jodie, St. Louis, MO 63105 (US)
(74) Representative: Goodall, Scott

(57) **Abstract**

The invention is directed to kits and methods that allow one to predict the risk for preterm labor in a pregnant woman. The inventors have discovered that various extracellular matrix components can be detected in cervical secretions. The quantification of certain extracellular matrix proteins found within cervical secretions can be used as a diagnostic for the biomechanical state of the cervix, which indicates whether preterm labor is likely. Some extracellular matrix components that can be found in cervical secretions include decorin, thrombospondins and matrix metalloproteinases.

## Description

### BACKGROUND OF THE INVENTION

### (1) Field Of the Invention

The invention relates generally to the diagnosis of an increased risk for pre-term delivery during pregnancy. More specifically, the invention relates to methods and kits useful in the prediction of preterm cervical softening.

### (2) Description Of the Related Art

Preterm labor, and subsequent delivery of premature infants, continues to remain a major cause of infant mortality and morbidity, as well as a serious economic burden on society. Premature softening of the uterine cervix during pregnancy results in premature cervical dilatation (opening) and effacement (thinning), and puts pregnant women at high risk of premature delivery. Currently there is no diagnostic method to predict premature dilatation of the cervix, and, therefore, the high-risk of preterm delivery can not be predicted in asymptomatic pregnant women.

While great strides have been made to decrease infant mortality, there has been little advancement in combating premature labor. Premature labor is now the leading factor in determining infant mortality. In North America, approximately one in ten pregnancies is complicated by preterm delivery. For example, the results of a multicenter trial spanning several years showed that infants born prematurely, i.e., between 20 and 36 weeks gestation, accounted for 9.6% of births (Cooper et at. Amer. J. Obstet. Gynecol. 168: 78, 1993). While there are many identified causes of preterm labor, such as infection and multiple gestation, for many patients the cause is unknown. There has been little advancement in recent decades to develop a method of accurately predicting and treating pre-term labor.

Babies born prematurely consume substantial health care resources. The hospitalization cost of preterm births is estimated to exceed $11 billion annually. The principle short term cost of these births is neonatal intensive care, time spent in the hospital by the parents, social workers and support staff for these parents, loss of earnings and increased travel expense. The long term cost of these premature births relates to the downstream effects of premature birth, such as long term health and developmental problems, increased risk of mental and physical handicap, which can impact both the child's and parent's earning potential.

Diagnosis of preterm labor and delivery has continued to be problematic. Currently there are two categories of preterm labor assessment; traditional diagnosis and biomarkers. While each has its advantages both are still relatively poor at differentiating false preterm labor. Traditional diagnosis includes evaluation of the frequency of uterine contractions, status of the membranes, dilatation and effacement of the cervix, and gestational age. Clinical preterm labor is defined as progressive cervical dilatation, effacement, or both, with regular contractions leading to birth before 37 weeks gestation.

Cervical changes are also traditional indicators of preterm delivery. Cervical changes include a reduction in effacement, and dilatation. When a cervix has a length of less than 1 cm and a dilatation of 2 cm or more, and regular contractions are occurring, preterm labor is diagnosed. However meta-analysis has shown that these criteria are only 50% predictive of preterm birth. Cervical length of less than 1.5cm before 30 weeks gestation was found to be a significant predictor of preterm labor. However, after 30 weeks this measurement is an unreliable predictor. Cervical effacement of more than 80% was also a strong predictive factor. However, assessment of contraction frequency, regularity, duration, and level of perceived pain does not reliably distinguish preterm labor.

### Biomarkers in Predicting Preterm Labor

Presently, several biomarkers are used to attempt to predict preterm labor sufficiently in advance to prevent preterm delivery. Those biomarkers include fetal fibronectin, salivary estriol, decidual proteins, and endocrine/paracrine markers. Two of these biomarkers have been thoroughly studied. Fetal fibronectin (Terao, et al. 5,650,394) and salivary estriol have been studied in some detail, and fetal fibronectin is commercially available. The fetal fibronectin test, currently on market as fFN (Adeza Biomedical /RossProducts Division Abbott Laboratories, Inc), is based on the detection of fetal fibronectin (a fetal-specific glycoform of fibronectin) from vaginal secretions. Current tests are based on the ELISA technique. The theory behind that test is that increased amounts of fetal fibronectin found in vaginal secretions signals the disturbance of the junction of the fetal membranes and the placental deciduas, and, therefore, an impending delivery. In symptomatic pregnant women, the test has limited effectiveness (58% sensitivity) to predict preterm delivery before the completion of 37 weeks gestation (33). However, the test has better predictive value for deliveries occurring within 1 week of the test (90% sensitivity) (34). Another value of this test is the ability to exclude the possibility of preterm delivery (85% specificity) (35). Thus, in asymptomatic pregnant women (low-risk of preterm delivery) the fetal fibronectin test has low sensitivity, but high specificity for predicting preterm delivery soon after the testing.

### Biomechanical Properties of Cervix

Cervical softening is a gradual physiological process that alters the mechanical properties of uterine cervix in preparation for dilatation and effacement. The uterine cervix, which is the connective tissue-rich sphincter at the overlapping boundary of the uterus and the cervix, functions to ensure the retention of the conceptus in the uterus during pregnancy. In the non-pregnant stage, or very early in pregnancy, the lumen of the cervix is very narrow, and the cervix is able to resist mechanical force to open it. By the end of the pregnancy, however, the cervix becomes highly extensible in order to dilate and efface to allow the passage of the newborn through the birth canal. The gradual increase in extensibility (a.k.a. cervical softening) begins early in pregnancy, and accelerates as pregnancy progresses toward the late stage in the third trimester (8-11). However, the cervix in this late stage is still able to function as a sphincter to retain the conceptus (12-14). The softening stage is followed by the effacement and dilatation stage, which is characterized by increased neutrophil infiltration (15, 16), increased prostaglandin production (17), increased nitric oxide production (18), increased protease activity with concomitant matrix degradation (19), and increased levels of cytokines in the cervix (16). Although the molecular bases of the effacement and dilatation stage are better understood, having this knowledge has not improved the management of the uterine cervix at the time of preterm delivery.

Remodeling of the stromal extracellular matrix is central to physiological cervical softening. Cervical stromal extracellular matrix is composed of types I and III collagens, proteoglycans, elastin, hyaluronan, and structural glycoproteins (4, 15, 16). This matrix can be considered a fiber-reinforced composite material with load-bearing types I and III collagen fibers embedded in a viscous matrix of proteoglycans and hyaluronan (20). Altering the alignment of the load-bearing elements, or their degree of dispersal in the matrix changes the mechanical properties of composite materials (21). Ample evidence supports the current concept that cervical stromal extracellular matrix is extensively remodeled during pregnancy. The collagen fibers and bundles are progressively dissociated and disorganized during cervical softening in mice (22), rats, (23, 24), guinea pig (25), sheep (26), and human (27, 28). Changes in collagen fibril-associated proteins, such as decorin and fibromodulin have been found to correlate with increased cervical extensibility (9, 29, 30). The loss in the degree of collagen crosslinking can also contribute to the increased disorganization of the cervical stroma during pregnancy (31).

According to observations made in inventor's laboratory, thrombospondin 2 plays a role in the maintenance and transformation of the biomechanical properties of the cervix. Thrombospondin 2 (TSP2) is a multimeric extracellular matrix glycoprotein that is capable of a wide variety of molecular interactions (48, 49), and is functionally distinct from TSP1 (50). Studies of TSP2-null mice have shown that a lack of TSP2 was associated with an altered morphology of fibrillar collagen fibers in skin and tendon (51), and with a corresponding decrease in tensile strength of those tissues.

Preliminary studies of a TSP2-null mouse, which were conducted in inventor's laboratory, demonstrated a premature cervical softening phenotype. As discussed in a recent paper from inventor's laboratory (32), by day 14 of pregnancy, the TSP2-null mice showed a 4.5-fold increase in cervical extensibility, and by day 18, a 6.1-fold increase, when compared to wild-type mice at the corresponding stages of pregnancy. Western blotting of wild-type cervices showed that TSP2 expression was detected on day 14 and day 18 of pregnancy, but not on day 10 or in non-pregnant cervices, indicating that TSP2 expression is correlated to the biomechanical status of the cervix. Additionally, immunohistochemical studies showed that levels of MMP2 in the uterine cervix increased 11-fold on day 14 and 19-fold on day 18 of pregnancy.

Despite all these investigations and observations, it still remains unclear what is the molecular basis of premature cervical softening and dilatation. This is due in large part to the difficulty of obtaining biopsies of human cervix from high-risk pregnancies, and to the absence heretofore of an animal model with spontaneous premature cervical softening. This lack of experimental systems has made it impossible to identify key molecular markers of cervical softening and to construct an appropriate diagnostic kit.

### References Cited

The following references are cited throughout this disclosure using the associated numerical identifiers. Applicant makes no statement, inferred or direct, regarding the status of these references as prior art. These references are incorporated herein by reference.
1. Mortality and Morbidity Weekly Reports 1999. Preterm singleton births - United States, 1986-199, 48:185-189.
2. Rogowski, J.A., 1998. The economics of preterm delivery. Prenat. Neonat. Med., 3:16-20.
3. Romero, R., Gomez, R., Mazor, M., Ghezzi, F., and Yoon, B.H. 1997. The preterm labor syndrome. In Preterm Labor. M.G. Elder, R. Romero, R.F.Lamont, editors, Churchill Livingstone, New York, 29-49.
4. Leppert, P.C. 1995. Anatomy and physiology of cervical ripening. Clin. Obstet. Gynecol., 38:267-279.
5. Jewelewicz, R. 1991. Incompetent cervix: pathogenesis, diagnosis and treatment. Semin. Perinatol. 2:156-161.
6. Colombo, D.F., and lams, J.D. 2000. Cervical length and preterm labor. Clin.Obstet. Gynecol. 43:735-745.
7. Althuisius S, Dekker GA, van Geijn HP. Cervical incompetence: a reappraisal of an obstetrics controversy. Obstet Gynecol Survey 2002; 57:377-387.
8. Cabrol, D. 1991. Cervical distensibility changes in pregnancy, term, and preterm labor. Semin. Perinatol. 2:133-139.
9. Kokenyesi R, Woessner JF, Jr. 1990. Relationship between dilatation of the rat uterine cervix and a small dermatan sulfate proteoglycan. Biol. Reprod. 42:87-97.
10. Harkness, M.L.R., and Harkness, R.D. 1959. Changes in the physical properties of the uterine cervix of the rat during pregnancy. J. Physiol. 148:524-547.
11. Kaga, N., Katsuki, Y., Kanikuma, C., Obata, M., Shibutani, Y., and Omata, S. 1996. Usefulness of a new tactile sensor for measurement of uterine cervical ripening in mice in a quantitative and noninvasive manner. Am. J. Obstet. Gynecol. 175:713-718.
12. Uldbjerg, N. 1989. Cervical connective tissue in relation to pregnancy, labour, and treatment with prostaglandin E2. Acta Obstet, Gynecol. Scand. Suppl. 148:4-40.
13. Thomson, A.J., Norman, J.E., and Greer, I.A. 1997. The cervix. In Preterm Labor. M.G. Elder, R. Romero, R.F.Lamont, eds., Churchill Livingstone, New York, 445-455.
14. Ludmir, J., and Sehdev, H.M. 2000. Anatomy and physiology of the uterine cervix. Clin. Obst. Gynecol. 43:433-439.
15. Junqueira, L.C.U., Zugaibe, M., Toledo, O.M.S., Krisztan, R.M., and Shigihara, K.M. 1980. Morphologic and histochemical evidence for the occurrence of collagenolysis and for the role of neutrophilic polymorphonuclear leukocytes during cervical dilatation. Am. J. Obstet. Gynecol. 138:273-281.
16. Rath, W., Winkler, M., and Kemp, B. 1998. The importance of extracellular matrix in the induction of preterm delivery. J. Perinat. Med. 26:437-441.
17. Gibb, W. 1998. The role of prostaglandins in human parturition. Ann. Med. 30:235-241.
18. Chwalisz, K., and Garfield, R.E. 1998. Role of nitric oxide in the uterus and cervix: implications for the management of labor. J. Perinat. Med. 26:448-457.
19. Winkler, M., and Rath, W. 1999. Changes in the cervical extracellular matrix during pregnancy and parturition. J. Perinat. Med. 27:45-60.
20. Aspden, R.M. 1988. The theory of fibre-reinforced composite materials applied to changes in the mechanical properties of the cervix during pregnancy. J. Theor. Biol. 130:213-221.
21. Hukins, D.W.L. 1984. Collagen orientation. In Connective Tissue Matrix, D.W.L. Hukins, editor. Verlag Chemie, Weinheim. 211-240
22. Leppi, T.J. and Kinnison, P.A. 1971. The connective tissue ground substance in the mouse uterine cervix: an electron microscopic study. Anat. Rec. 170:97-118.
23. Yu, S.Y., Tozzi, C.A., Babiarz, J., Leppert, P.C. 1995. Collagen changes in rat cervix in pregnancy - polarized light microscopic and electron microscopic studies. Proc. Soc. Exp. Biol. Med. 209:360-368.
24. Saito, Y., Takahashi, S., and Maki, M. 1981. Effects of some drugs on ripening of uterine cervix in castrated and pregnant rats. Tohoku J. Exp. Med. 133:205-220.
25. Hegele-Hartung, C., Chwalisz, K., Beier, H.M., and Elger, W. 1989. Ripening of the uterine cervix of the guinea-pig after treatment with the progesterone antagonist onapristone (ZK 98.299): and electron microscopic study. Human Reprod. 4:369-377.
26. Ellwood, D.A., Anderson, A.B.M., Mitchell, M.D., Murphy, G., and Turnbull,A.C. 1981. Prostanoids, collagenase and cervical softening in sheep. In The Cervix in Pregnancy and Labour. D.A. Ellwood and A.B.M. Anderson editors, Churchill Livingstone, London, 57-73.
27. Buckingham, J.C., Selden, R., and Danforth, D.N. 1962. Connective tissue changes in the cervix during pregnancy and labor. Ann. New York Acad. Sci. 97:733-742.
28. Theobald, P.W., Rath, W., Kuhnle, H., and Kuhn, W. 1982. Histological and electron-microscopic examinations of collagenous connective tissue of the non-pregnant cervix, pregnant cervix, and the pregnant prostaglandin-treated cervix. Arch. Gynecol. 231:241-245.
29. Kokenyesi, R., and Woessner, J.F., Jr. 1991. Effects of hormonal perturbations on the small dermatan sulfate proteoglycan and mechanical properties of the uterine cervix of late pregnant rats. Connective Tissue Res. 26:199-205
30. Westergren-Thorsson, G., Norman, M., Bjornsson, S., Endresen, U., Stjernholm, Y., Ekman, G., and Malmstrom, A. 1998. Differential expressions of mRNA for proteoglycans, collagens and transforming growth factor-beta in the human cervix during pregnancy and involution. Biochim. Biophys. Acta 1406:203-213.
31. Glassman, W., Byam-Smith, M., and Garfield, R.E. 1995. Changes in rat cervical collagen during gestation and after antiprogesterone treatment as measured in vivo with light-induced autofluorescence. Am. J. Obstet. Gynecol. 173:15501556.
32. Kokenyesi, R., Armstrong, L.C., Agah, A., Artal, R., and Bornstein, P. 2004. Thrombospondin 2 deficiency in pregnant mice results in premature softening of the uterine cervix. Biol Reprod. 70:385-390
33. Revah A, Hannah ME, Sue-A-Quan AK. 1998. Fetal fibronectin as a predictor of preterm birth: an overview. Am J Perinatol. 15:613-621.
34. Peaceman AM, Andrews WW, Thorp JM, Cliver SP, Lukes A, lams JD, Coultrip L, Eriksen N, Holbrook RH, Elliott J, Ingardia C, Pietrantoni M. 1997. Fetal fibronectin as a predictor of preterm birth in patients with symptoms: a multicenter trial. Am J Obstet Gynecol.177:13-18.
35. Leitich H, Kaider A. 2003. Fetal fibronectin--how useful is it in the prediction of preterm birth? BJOG. 110 Suppl 20:66-70.
36. lams JD, Goldenberg RL, Meis PJ, Mercer BM, Moawad A, Das A, Thom E, McNellis D, Copper RL, Johnson F, Roberts JM. 1996. The length of the cervix and the risk of spontaneous premature delivery. National Institute of Child Health and Human Development Maternal Fetal Medicine Unit Network. N Engl J Med. 334:567-572.
37. lams JD. 2003. Prediction and early detection of preterm labor. Obstet Gynecol. 101:402-412.
38. Garfield RE, Chwalisz K, Shi L, Olson G, Saade GR. 1998. Instrumentation for the diagnosis of term and preterm labour. J Perinat Med. 26:413-436.
39. Fittkow CT, Shi SQ, Bytautiene E, Olson G, Saade GR, Garfield RE. 2001. Changes in light-induced fluorescence of cervical collagen in guinea pigs during gestation and after sodium nitroprusside treatment. J Perinat Med. 29:535-543.
40. Bukowski R, Mackay L, Fittkow C, Shi L, Saade GR, Garfield RE. 2001. Inhibition of cervical ripening by local application of cyclooxygenase 2 inhibitor. Am J Obstet Gynecol. 184:1374-1378.
41. Heine RP, McGregor JA, Goodwin TM, Artal R, Hayashi RH, Robertson PA, Varner MW. 2000. Serial salivary estriol to detect an increased risk of preterm birth. Obstet Gynecol. 96:490-497.
42. Ellis MJ, Livesey JH, Inder WJ, Prickett TC, Reid R. 2002. Plasma corticotropin-releasing hormone and unconjugated estriol in human pregnancy: gestational patterns and ability to predict preterm delivery. Am J Obstet Gynecol. 186:94-99.
43. Goldenberg RL, lams JD, Mercer BM, Meis PJ, Moawad A, Das A, Miodovnik M, Vandorsten PJ, Caritis SN, Thurnau G, Dombrowski MP; Maternal-Fetal Medicine Units Network. 2001. The Preterm Prediction Study: toward a multiple-marker test for spontaneous preterm birth. Am J Obstet Gynecol. 185:643-651.
44. Leppert, P.C., Kokenyesi, R., Klemenich, C.A., and Fisher, J. 2000. Further evidence of a decorin-collagen interaction in the disruption of cervical collagen fibers during rat gestation. Am. J. Obstet. Gynecol. 182:805-812.
45. Kobayashi H, Sun GW, Terao T. 1999. Immunolocalization of hyaluronic acid and inter-alpha-trypsin inhibitor in mice. Ce// Tissue Res. 296:587-597.
46. Chapdelaine P, Vignola K, Fortier MA. 2001. Protein estimation directly from SDS-PAGE loading buffer for standardization of samples from cell lysates or tissue homogenates before Western blot analysis. Biotechniques 31:478- 482.
47. Kokenyesi R, Murray KP, Benshushan A, Huntley ED, Kao MS. 2003. Invasion of interstitial matrix by a novel cell line from primary peritoneal carcinosarcoma, and by established ovarian carcinoma cell lines: role of cell-matrix adhesion molecules, proteinases, and E-cadherin expression. Gynecol Oncol 89:60-72.
48. Adams, J.C. Thrombospondins: multifunctional regulators of cell interactions. Annu Rev Cell Dev Biol 2001; 17:25-51.
49. Bornstein, P., Armstrong, L.C., Hankenson, K.D., Kyriakides, T.R., and Yang, Z. 2000. Thrombospondin 2, a matricellular protein with diverse functions. Matrix Biol 19:557-568.
50. Bornstein, P. Thrombospondins as matricellular modulators of cell function. 2001. J Clin Invest 107:929-934.
51. Kyriakides, T.R., Zhu, Y.H., Smith, L.T., Bain, S.D., Yang, Z.T., Lin, M.T., Danielson, K.G., lozzo, R.V., LaMarca, M., McKinney, C.E., Ginns, E.I., and Bornstein, P. 1998. Mice that lack thrombospondin-2 display connective tissue abnormalities that are associated with disordered collagen fibrillogenesis, an increased vascular density, and a bleeding diathesis. J Cell Biol 140:419-430.
52. Kokenyesi, R., and Woessner, J.F., Jr. 1989. Purification and characterization of a small dermatan sulfate proteoglycan implicated in the dilatation of the rat uterine cervix. Biochemical Journal 260:413-419.

### SUMMARY OF THE INVENTION

The inventor has made the surprising and important discovery that a collagen fibril associated protein is involved in changes in the cervix during pregnancy in humans. Thus the inventor envisions that collagen fibril associated proteins, glycosaminoglycans, glycosaminoglycan-associated proteins, and other extracellular matrix components, including structural as well as remodeling and regulatory components (e.g., matrix metaloproteinases ("MMPs")) can serve as biomarkers in the prediction of preterm cervical softening and the risk for preterm labor in mammals, including humans.

Thus, the invention is directed to a method of predicting preterm labor in a pregnant woman comprising the steps of obtaining a lower uterine/cervical sample from the pregnant woman, assessing the level of an extracellular matrix component (a.k.a. biomarker) in the sample, comparing the level of the extracellular matrix component to a control standard or a baseline standard, and predicting the risk for preterm cervical softening and preterm labor. The lower uterine/cervical sample can be a lower uterine tissue biopsy, a cervical secretion, or a vaginal secretion. Preferably, the sample is a cervical secretion, which can be obtained non-invasively with a swab.

Preferred extracellular matrix components include thrombospondin 2 ("TSP2"), hyaluronic acid ("HA"), matrix metalloproteinase 2 ("MMP2"), matrix metalloproteinase 12 ("MMP12"), decorin, lumican and fibromodulin. More preferred extracellular matrix components include TSP2, HA, MMP2 and MMP12. Table 1 provides a list of biomarkers useful in the assessment of the state of cervical softening and the risk for preterm cervical softening.

The control standard can be a standard amount or a dilution series of an extracellular matrix component. A baseline standard can be a protein or other biomolecules that is constitutively (i.e., not specifically associated with progression of pregnancy) present in lower uterine cells, such as for example actin, glyceraldehyde phosphodehydrogenase ("GAPDH") and the like. The extracellular matrix component can be detected and quantified using any specific biomolecular recognition assay known in the art, such as an antibody assay, an aptamer assay, a ligand-receptor assay and an allosteric enzyme activity assay. A standard antibody assay can be ELISA, western blotting, dot-blotting, RIA, antibody sandwich assay, antibody capture assay, antigen capture assay and the like. One or more biomarkers can be assessed in the method. In an alternative aspect of this embodiment, multiple biomarkers can be assessed and a diagnostic decision can be made based upon a multiplicity of signals.

### Table 1: Useful Biomarkers for Assessing Cervical Softening / Risk for Preterm Delivery

Lumican, fibromodulin, decorin, keratocan, PRELP biglcan, mimecan, serglycin, perlecan, agrin, versican, link proteins, CD44, TSG-6, bikunin, inter alpha trypsin inhibitor, hyaluranan synthases, hyaluronidases, elastin, fibulins, fibrillins, tenascin X, heparan sulfate, keratan sulfate, chondroitin sulfate, dermatan sulfate, heparanase, lysyl oxidase, type V collagen, type VI collagen, type XII collagen, type XIV collagen, type XVIII collagen, type XX collagen, calreticulin, CD47, syndecans, glypicans, lipoprotein receptor-like protein (LRP), ADAM and ADAMTS proteases, E-selectin, L-selectin, platelet factor 4, transforming growth factors beta 1, 2 and 3 (TGF-β), epidermal growth factor (EGF), keratinocyte growth factor (KGF), vascular endothelial growth factor (VEGF), hepatocyte growth factor (HGF), fibroblast growth factors (FGF) 1 and 2, and platelet derived growth factor (PDGF).

In yet another embodiment, the invention is directed to a preterm labor test kit which can detect and quantify a biomarker that is associated with risk for preterm cervical softening and preterm labor. In one embodiment, the kit comprises in a first part, which is used in a clinical setting, a swab to obtain vaginally a cervical secretion sample from a pregnant woman, and a buffer. The kit comprises in a second part, which is used in a diagnostic setting, a mechanical support (e.g., membrane, test tube, Petri plate, multiwell plate, or the like), and a first agent that specifically recognizes a biomarker. In a preferred embodiment, the kit comprises in the second part a second agent, a signaling molecule, wherein the signaling molecule is linked to the second agent, and optionally a biomarker to serve as an internal control. An agent (whether first or second) may be an aptamer, a ligand, a receptor, a substrate of an enzyme, a product of an enzyme, an allosteric regulator, an antibody, or a fragment of any thereof, which can bind specifically to the biomarker. In the case of a second agent, the second agent can specifically bind to either a first agent, a biomarker, or both. A preferred first agent is an antibody or antibody fragment that binds specifically to a biomarker. A preferred biomarker is one or more of the following: Decorin, TSP2, MMP2, MMP12 and HA. A signaling molecule can be a luminescent moiety (e.g., fluorescent tag), an enzyme (e.g., HRP or alkaline phosphatase), a radiolabel (e.g., ¹²⁵I), high affinity moiety (e.g., biotin), or the like. In another aspect of the kit embodiment, the kit may comprise, in a single part, a swab to obtain vaginally a cervical secretion sample from a pregnant woman, a buffer, a mechanical support, a first agent, a second agent, and a control biomarker (supra).

In another embodiment of the present invention, the kit for the quantification of a biomechanical associated marker is accomplished by measuring the amount of nucleic acid molecules coding for a biomechanical associated marker in the cervical secretion sample.

In one embodiment of the invention, the nucleic acid molecule to be measured is DNA.

In another embodiment of the present invention, the nucleic acid molecule to be measured is RNA.

In one embodiment of the invention, the nucleic acid molecule to be measured is cDNA.

In yet another embodiment the invention is drawn to a method for determining the risk for preterm labor in a pregnant patient, the method comprising the steps of (a) obtaining a cervical secretion sample from a patient, preferably using a swap, (b) placing the swap containing the cervical secretion sample into a buffer, (c) subjecting the buffer, which contains various biomarkers, to a quantitative immunoassay, (d) determining the relative amounts of one or more biomarkers associated with cervical softening in the sample, and (e) and assessing the risk for preterm labor, based upon those relative amounts. By relative amount of biomarker, what is meant is that the amount of biomarker in a sample is quantified relative to the amount of a baseline control marker in the sample. The baseline control marker is a marker whose level does not change, or changes very little over the course of pregnancy progression. In a preferred embodiment, the sample is taken in a clinical environment, e.g. the doctor's office, placed into the buffer, which helps to maintain the integrity of the biomarkers and baseline control markers in the sample, and transported to a laboratory testing facility. At the laboratory facility, the sample is processed, e.g., as in a quantitative immunoassay or similar-in-principle biomolecular assay, and the one or more biomarker is quantified relative to one or more baseline control marker. The risk for preterm labor is determined based upon the relative amount of one or more biomarker and that risk is communicated to the patient and/or patient's health care worker.

In a preferred aspect of both the kit and method embodiments, an increase in MMP12, Decorin or TSP2, either alone or in combination with one or more of another biomarker, significantly above baseline levels, indicates an increased risk for preterm labor. Baseline levels means the average normalized optical density (in arbitrary units as described herein [infra]) of any one or more of the biomarkers expressed in cervical secretions of women who deliver at term. Optical density may be expressed in arbitrary units. Preferably, the preterm positive levels are at least one standard deviation above the normal baseline levels of at least one biomarker. By any one or more, what is meant is that TSP2 alone or in combination with either Decorin, MMP12, or any other biomarker set forth in Table 1 (infra) or multiple combinations thereof, can be quantified and used to determine risk for preterm labor.

While it is envisioned by the inventors that the cervical samples can be obtained at any point during pregnancy, preferred times to collect a sample include 22 weeks and 26 weeks of pregnancy. More samples at other times can be collected to monitor those cases considered to be of greater risk of preterm labor. In another aspect, since the invention is directed to ascertaining to some degree or other the biomechanical status of the cervix, the test can be provided to a patient prior to induction of labor to assess whether the cervix has the capacity to allow delivery.

The inventor envisions that the above described cervical biomarker test may be useful in other pregnancy-related assessment situations. For example, this biomarker assay can be used to monitor the effectiveness of therapy to inhibit (stop or slow down) cervical softening and/or uterine contractions. Also the biomarker assay can be used to assess the status of the cervix at anytime during pregnancy (but it shall not be limited to pregnancy situations only) For example, the biomarker assay can be used to determine whether the cervix is sufficiently ready to deliver a baby, such as prior to the administration of pitocin, thereby helping to mitigate downstream problems. The skilled artisan, to which this invention is directed, may reasonable see the applicability of this invention to myriad obstetrical and gynecological tests.

### BRIEF DESCRIPTION OF THE DRAWINGS

Figure 1 depicts a graph showing quantification of in situ staining levels of thrombospondin 2 in lower uterine tissues from women at various stages of pregnancy in weeks of gestation.
Figure 2 depicts a graph showing quantification of in situ staining levels of decorin in lower uterine tissues from women at various stages of pregnancy in weeks of gestation.
Figure 3 depicts western blot strips showing the presence of protein extracellular matrix components in human cervical secretion samples.
Figure 4 depicts dot blot strips showing the presence of hyaluronic acid in human cervical secretion samples.

Figure 5 depicts a bar graph of densitometry values for various extracellular matrix components of human cervices as detected immunologically by cervical swab samples obtained from pregnant humans. TSP2 = thrombospondin 2, ADAMTS4 = Alzheimer's disease associated thrombospondin type 1 motif 4, MMP3 = matrix metalloproteinase 3, MMP1 = matrix metalloproteinase 1, MMP8 = matrix metalloproteinase 8.

Figure 6 depicts a bar graph of optical densitometry values for thrombospondin-2 (TSP2) levels for patients who had preterm deliveries versus those that delivered at term. The optical densitometry values were normalized to total protein levels.

Figure 7 depicts a bar graph of optical densitometry values for Decorin levels for patients who had preterm deliveries versus those that delivered at term. The optical densitometry values were normalized to total protein levels.

Figure 8 depicts a bar graph of optical densitometry values for MMP-12 levels for patients who had preterm deliveries versus those that delivered at term. The optical densitometry values were normalized to total protein levels.

### DESCRIPTION OF A PREFERRED EMBODIMENT

The inventors made the surprising discovery that extracellular matrix components (e.g., thrombospondin 2, MMP2, MMP12, HA and decorin), which are involved in modulating the biomechanical properties of cervix and lower uterine segment in mammals, (1) are expressed differentially during the progression of pregnancy in cervix and lower uterine segments of mammals, in particular humans, and (2) are capable of being detected in cervical secretions. The inventors envision that these and other extracellular matrix components, as well as other biomarkers that are listed in table 1, can serve as biomarkers in the prediction of the risk for preterm labor in pregnant women and in the assessment of the readiness of a cervix for delivery of a baby. Thus the invention is directed to methods of diagnosing and predicting the risk for preterm cervical softening and concomitant preterm labor in women, as well as the assessment of the readiness of a cervix for delivery, and kits that are useful in the diagnosis and prediction of preterm cervical softening and concomitant preterm labor in women, and in the assessment of the readiness of a cervix for delivery. The method and kit comprise the use of one of more biomarker(s), which is/are demonstrated to be or are reasonably expected to be associated with a change in the biomechanical properties of the cervix and/or lower uterine segment. The biomarkers are preferably but not necessarily limited to extracellular matrix components selected from thrombospondin 2 (TSP2), decorin, fibromodulin, lumican, hyaluronan (HA), matrix metalloproteinase 2 (MMP2, a.k.a. elastase), and matrix metalloproteinase 12 (MMP12).

In one embodiment of the present invention, a method for determining the risk of a patient going into preterm labor comprises (a) obtaining a cervical secretion sample from a pregnant patient; (b) detection the presence of a biomechanical associated marker in the cervical secretion sample, wherein the biomechanical associated marker is selected from the group consisting of thrombospondin 2 and decorin and combinations thereof; and (c) correlating an increased level of the biomechanical associated marker, as compared to a normalized baseline marker level, to an increased risk of preterm labor.

In another embodiment of the present invention, the method for determining the risk of a patient going into preterm labor further comprising detecting the presence of matrix metalloproteinase-12 and correlating an increased level of matrix metalloproteinase-12 , as compared to a normalized baseline marker level, to an increased risk of preterm labor.

In another embodiment of the present invention, the method of determination of the risk for preterm labor further comprises the determination fo an appropriate treatment protocol for the patient, which includes at least one of the following treatments: i) physical relocation of the patient; ii) administration of drug therapy to the woman; iii) recommendation of a behavioral change to the patient; and iv) closer monitoring of the patient.

In one embodiment of the invention, the drug therapy to be administered to the patient is progesterone or derivatives of progesterone. Examples of derivatives of progesterone include, but are not limited to, micronized progesterone, injected 17-OH progeseterone and oral medroxyprogesterone acetate. The progesterone can be delivered to the patient vaginally, sublingually or rectally.

In another embodiment of the present invention, the drug therapy to be administered to the patient is a compound that stimulates or blocks uterine contractions. Preferably, the compound that stimulates uterine contractions is pitocin or oxytocin, and the compound that inhibits uterine contractions is an oxytocin analogue.

In another embodiment of the present invention, the method of determination of the risk for preterm labor, specifically the detection of a biomechanical associated marker, is accomplished by measuring the amount of nucleic acid molecules coding for the biomechanical associated marker in the cervical secretion sample.

In one embodiment of the invention, the nucleic acid molecule to be measured is DNA.

In another embodiment of the present invention, the nucleic acid molecule to be measured is RNA.

In one embodiment of the invention, the nucleic acid molecule to be measured is cDNA.

As discussed herein (supra), the methods and kits can utilize an antibody- or aptamer-based assay to determine the relative amounts in a biological sample of a biomarker that reflects the condition of the lower uterine segment or cervix of a pregnant woman. The biological sample can be a tissue biopsy (cervix or lower uterine segment), a cervical secretion, or a vaginal secretion. The sample can be taken from any pregnant mammal, preferably a human, at any one time or multiple times during pregnancy. In a preferred embodiment for humans, a cervical secretion sample may be taken by a skilled clinician using a swab at 22, 24, 26, 28, 30, and 32 weeks of gestation, times that ensure adequate time to intervene to protect early delivery. However, the skilled obstetrician in the practice of this invention would readily recognize that this invention has applicability at multiple other times during pregnancy to assess the molecular-biomechanical status of the cervix.

The following examples describe a kit and method, which use a cervical secretion sample. The skilled artisan will readily appreciate that myriad biomolecular detection methods may be employed in the practice of this invention. For a description of antibody-based methods of detection, see Harlow and Lane, Antibodies: A Laboratory Manual, CSHL Press, 1988, Cold Spring Harbor, NY, which is herein incorporated by reference. For a description of aptamer-based methods of detection, see Heyduk and Heyduk, "Nucleic acid-based fluorescence sensors for detecting proteins," Anal Chem. 2005 Feb 15;77(4):1147-56, which is herein incorporated by reference.

### Example 1: Kit and Method to Assess Biomarkers in Cervical Secretion Sample

Cervical secretions are collected with Dacron swabs. The exposed ectocervical surface and the posterior fornix are swabbed, and the swabs are placed into a collecting vial containing physiological saline and preservatives. The sample collection is very similar to that used for other routine assays (fetal fibronectin) that use cervical secretions as the starting material.

A concentrated detergent solution is added to the collecting vial, and the swabs are agitated in order to achieve complete release of molecules into the solution. The swabs are removed, and the sample solutions are clarified by brief centrifugation. The clarified samples and aliquots of purified standards (various concentrations of TSP2, HA, MMP2, and MMP12) are loaded into a dot-blotting apparatus, and the solution is drawn through a high-affinity protein-binding membrane (PVDF). The resulting protein spots will be used for protein quantification and for biomarker quantification.

The membrane comprising the sample protein spots are treated with a blocking solution (e.g., 5% dry milk in Tris-buffered saline) for 30 minutes. The membranes are incubated with the primary antibodies specific for TSP2, MMP2 and MMP12. In the case of HA detection, a biotinylated hyaluronan-binding protein (from Calbiochem) is used instead of primary antibody. The antibodies and the hyaluronan-binding protein can be dissolved in 5% dry milk solution. Incubation is for about 12 hours at 4°C, or for shorter times at room temperature or 37°C. After the incubation the membranes are washed with Tris-buffered saline, and then incubated with peroxidase-conjugated secondary antibodies (for TSP2, MMP2 and MMP12), or peroxidase-conjugated avidin (for HA). After this incubation the membranes are washed several times with Tris-buffered saline, and the color development is done using diaminiobenzidine substrate. The color intensity is quantified using densitometry.

To generate a standard curve, known amounts of TSP2, MMP2, MMP12, and HA are spotted in increasing quantities on a membrane. The detection method is identical to that described in the previous section (supra). Standard curves will be derived by plotting the staining intensity against the amount of biomarker. Optical densities of the stained spots on the spot-ELISA assays will be determined by analyzing the digitized images of the ELISA membranes using a program such as the Scion 4.2 program. The standard curves for TSP2, HA, MMP2 and MMP12 allows the practitioner to determine the absolute amount of a biomarker in a sample. The amount of a biomarker is normalized to the standard curve control.

The sensitivity of the assay can be increased by using a fluorescence readout as an alternative to a colorimetric read-out. This fluorescence-based readout is based on the general format of the spot-ELISA described above (supra), wherein a fluorescence-generating substrate is used instead of the diaminiobenzidine substrate. The equipment for detection of the signal and the densitometric quantitation is performed essentially as described for the colorimetric approach.

The whole protein content of the samples can be determined by the method of Chapdelaine et al (46). This method is based on using amido black solution on membrane-bound proteins, and quantifying the color intensity by densitometry. This method has been successfully employed to cells in culture (47).

### Example 2: Biomarker Expression in Lower Uterine Segment

The maintenance of pregnancy to term requires the coordinated function of the uterus and the uterine cervix. The cervix is a sphincter-like structure composed mainly of connective tissue that undergoes substantial remodeling during pregnancy to allow dilatation at term (Leppert, P.C, 1995, Clin Obstet Gynecol, 38:267-79). Abnormal remodeling of the human cervix is thought to cause "incompetent cervix", or milder forms of premature cervical softening and dilatation. These cervical disorders are significant contributors to premature deliveries.

In order to determine the contribution of individual components of connective tissues to cervical function, inventor chose to study cervical properties of mice deficient in individual proteins of connective tissue. Several collagen fibril-associated proteins (decorin, lumican, fibromodulin, thrombospondin 2) have been shown to regulate morphology of collagen fibrils, and mechanical properties of skin in mice. The objective was to gain a better understanding of the molecular basis of premature cervical softening by determining the characteristics of the cervix of the thrombospondin 2-null (TSP2-null) mouse (Kyriakides, T.R., et al, 1998, J Cell Biol, 140:419-30).

The extensibility of the cervix was determined by creep testing, and the cervix of TSP2-null mice showed a 4.5-fold increase by day 14 of pregnancy, and a 6.1-fold increase by day 18 of pregnancy, when compared to wild-type mice (Kokenyesi, R., et al, 2004, Biol Reprod, 70:385-90, which is herein incorporated by reference). The extensibility of the TSP2-null cervix was similar to that of the wild-type cervix in non-pregnant mice and on day 10 of pregnancy. In agreement with the timing of the biological defect, the expression pattern of TSP2 (determined by Western blotting) showed that TSP2 was expressed in the cervix of mice on days 14 and 18 of pregnancy, but not on day 10 or at the non-pregnant stage. In addition to an increased extensibility, a defective biological function of the TSP2-null cervix was also demonstrated in the TSP2-null mice. Mifepristone treatment of mice on day 15 of pregnancy resulted in an accelerated delivery in TSP2-null mice compared to wild-type mice. An increased level of matrix metalloprotease 2 (MMP2) immunostaining was found in the TSP2-null cervix on day 14 and day 18 of pregnancy. Correspondingly, increased MMP2 activity was detected by gelatin zymography in extracts of TSP2-null cervix on day 14 of pregnancy. No MMP9 activity was seen by gelatin zymography. Casein zymography showed no change in caseinolytic activity of the TSP2-null cervix on day 14 of pregnancy. Inventor also evaluated immunohistochemical staining of MMP3 (stromelysin), MMP7 (matrilysin), MMP13 (the major mouse collagenase), and cathepsin G, because these are proteases known in the art to be involved in the remodeling of connective tissues. Densitometric analysis of stained membranes showed no significant change in the staining intensity of any of these proteases in the TSP2-null cervix on day 14 of pregnancy. Inventor failed to detect degradation products of decorin and lumican (two MMP substrates expressed abundantly in the cervix) in the TSP2-null cervix. The expression of tissue inhibitors of metalloproteases (TIMPs) on day 14 of pregnancy was also studied. TIMPS 1, 2, and 4 were detected by immunohistochemical staining; however, densitometric analysis showed the absence of significant changes in the staining intensity. TIMP3 was not detected in the cervix.

In order to gain a better understanding of the type and magnitude of the ultrastructural alterations in the TSP2-null cervix, applicant used quantitative morphometry to characterize the ultrastructural features of the collagenous stromal matrix. A standardized sampling protocol was developed to obtain an unbiased representation of the matrix structures on the transmission electron microscopic images. In the TSP2-null cervix on day 14 of pregnancy, applicant observed an increased collagen fibril diameter, altered distribution of fibril diameters, a decrease in the cross-sectional area of collagen bundles, a decreased packing density of collagen fibrils within the bundles, an accumulation of electron lucent particles in between the collagen fibrils, and an increased proportion of the disorganized collagenous matrix.

The presence of hyaluronan in the TSP2-null cervix was examined by immunohistochemical techniques. The hyaluronan staining was observed more in the stroma , and less in the epithelium. Densitometric analysis showed a significant increase in the hyaluronan staining intensity on day 14 and day 18 of pregnancy, but not on day 10. From these data, the skilled artisan may reasonably conclude that expression TSP2 in the mouse uterine cervix is important for the maintenance of tissue integrity and resistance to mechanical force. Thus, the TSP2-null mouse is the first animal model that allows for the study of spontaneous, premature cervical softening. The premature cervical softening seen in the TSP2-null mouse on day 14 of pregnancy correlates with extensive alterations in the ultrastructure of the stromal matrix and increased MMP2 and hyaluronan levels, while many other protease/protease inhibitor systems are not affected. These findings suggest that the premature cervical softening is brought about by altered balance of assembly/disassembly of collagen bundles with limited involvement of proteolytic action. This data demonstrates that the absence of a single collagen fibril-associated protein can significantly alter cervical extensibility, suggesting that TSP2 and other collagen fibril-associated proteins (such as decorin or lumican) fulfill specific, non-redundant roles in regulation of the integrity of connective tissues. While not wishing to be bound by theory, applicant reasonably speculates that defective expression of any collagen fibril-associated protein or regulator of the cervical extracellular matrix may lead to defective cervical functioning in pregnant women.

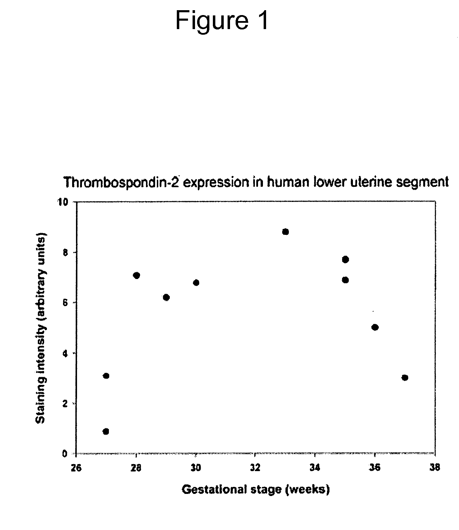

Because TSP2 has not been studied in the human reproductive tract, the inventor carried out an immunohistochemical study on the expression pattern of TSP2 in human lower uterine segment. TSP2 staining was seen in the connective tissue-rich areas located between smooth muscle cells of all specimens ranging from 27 to 36 weeks of gestation. There was little staining of the smooth muscle cells themselves. Staining intensity of TSP2 peaked at 33-35 week of pregnancy, and the staining was less intense at 27-31 weeks, and 36-37 weeks (see figure 1, which depicts quantified TSP2 staining from human lower uterine samples versus gestational stage in weeks.)

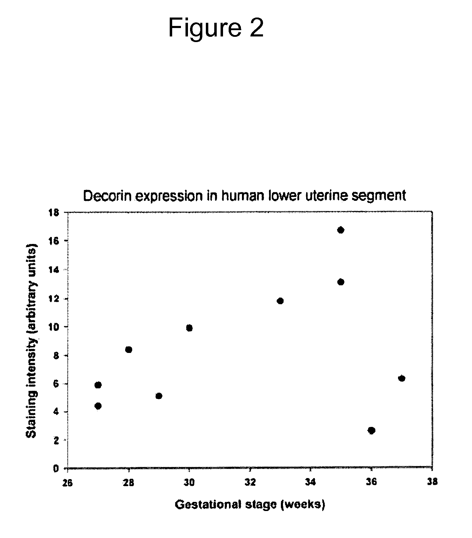

Lower uterine segment specimens were obtained by a protocol approved by the Institutional Review Board of Saint Louis University. Specimens were obtained from patients undergoing Caesarian section for indications other than premature labor. The range of gestational ages was 27 to 37 weeks. The specimens were fixed in buffered formalin, and processed for immunohistochemistry. A monoclonal antibody (from BD Biosciences) specific for TSP2 was used at 1:50 dilution, followed by a horseradish peroxidase-conjugated second antibody. Immunohistochemical staining for decorin was performed essentially as described (44), except that a polyclonal sheep anti-human decorin antibody (from US Biological) was used at 1:50 dilution. Images were recorded by Nikon Eclipse E400 microscope equipped with a digital camera. The staining pattern for decorin was used as a reference, because decorin expression in the cervical extracellular matrix has been repeatedly demonstrated. Both TSP2 (Figure 1) and decorin (Figure 2) were detected at locations of matrix accumulations between muscle fibers. The smooth muscle cells showed very weak staining for TSP2 and decorin.

### Example 3: HA and MMP12 as Cervical Biomarkers

Upon further study of the animal model of spontaneous, premature cervical softening, inventor discovered elevated hyaluronic acid (HA), MMP2 and MMP12 immunostaining in the prematurely softened cervices. Thus, inventor envisions that increased levels of HA, MMP2 and MMP12 in the cervix are useful as biomarkers for premature cervical dilatation.

HA levels were studied using histochemistry following the method of Kobayashi and co-workers (45). This detection method is based on the specific binding of biotinylated HA-binding protein to HA and the subsequent visualization of hyaluronic acid-binding protein by avidin peroxidase. Controls were performed by omitting the biotinylated hyaluronic acid-binding protein from the staining sequence. The specificity of this technique was established by pre-treating cervical sections with 10 mU of Streptomyces hyaluronidase at 37°C for 1 hour. Such pre-treatment completely eliminated staining of the sections indicating that the hyaluronic acid-binding protein is specific for HA in mouse cervical sections. Histomorphometry (using the Image J program) was used to quantify the staining intensity. Three cervices were studied for each genotype and each gestational stage; at least 6 high-power fields from the external os area of each cervix were used for image analysis.

In wild-type mice the staining intensity for HA increased from 4.2 units (+/-0.4 SEM) on day 10 of pregnancy to 6.6 units (+/-0.8 SEM) on day 14 of pregnancy, and to 13.5 units (+/-1.1 SEM) on day 18 of pregnancy. In the TSP2-null mice the staining intensity for HA increased from 3.8 units (+/-0.4 SEM) on day 10 of pregnancy to 11.9 units (+/-1.4 SEM) on day 14 of pregnancy, and to 17.4 units (+/-1.0 SEM) on day 18 of pregnancy. There was no detectable difference (p<0.25) between the HA staining intensity of wild-type and TSP2-null cervix on day 10 of pregnancy. However, the HA staining intensity appeared to be significantly higher (p<0.01) in the TSP2-null cervix on day 14 and day 18 of pregnancy.

MMP12 expression was also studied by immunohistochemical staining. Cervices from timed pregnant females on day 14 of pregnancy were studied. The primary (MMP12-specific antibody) was purchased from Santa Cruz Biotechnology, Inc. The staining intensity for MMP12 was 10.5 units (+/-0.9 SEM) in the wild-type cervices, and increased to 16.5 units (+/-0.5 SEM) in the TSP2-null cervices. Histomorphometry (using the Image J program as described for the proteases) was used to quantify the staining intensity. Three cervices were studied for each genotype and each gestational stage; at least 6 high-power fields from the external os area of each cervix were used for image analysis.

### Example 4: Human Cervix / Lower Uterine Segment

The expression of TSP2 in human lower uterine segment (a tissue that represent a transition from the uterus to the cervix) was determined. Lower uterine segment specimens were obtained by a protocol approved by the Institutional Review Board of Saint Louis University. Specimens were obtained from patients undergoing Caesarian section for indications other than premature labor. The range of gestational ages were 27 to 37 weeks. The specimens were fixed in buffered formalin, and processed for immunohistochemistry. A monoclonal antibody (from BD Biosciences) specific for TSP2 was used at 1:50 dilution, followed by a horseradish peroxidase-conjugated second antibody. Immunohistochemical staining for decorin was performed essentially as described (44), except that a polyclonal sheep anti-human decorin antibody (from US Biological) was used at 1:50 dilution. The staining pattern for decorin is used as a reference, because decorin expression in the cervical extracellular matrix has been repeatedly demonstrated. The images were recorded by Nikon Eclipse E400 microscope equipped with a digital camera.

Both TSP2 and decorin were detected at locations of matrix accumulations between muscle fibers. The smooth muscle cells showed very weak staining for TSP2 and decorin. Thus, having determined that TSP2 is present in human uterine cervix, the skilled artisan may reasonably expect that TSP2 is likely also to be present in cervical secretions (infra).

Next, biopsy specimens representing various gestational stages were stained for TSP2, and the staining intensities from the slides were quantified from the digitized images using the Image J program (a variant of the NIH Image program). Six high-power fields were evaluated for each specimen. The staining intensity of TSP2 showed an increasing trend from 27 weeks of gestation to 35 weeks of gestation, and then a decreasing trend during 36 and 37 weeks of gestation (Figure 1).

### Example 5: Biomarkers in Cervical Secretions

The candidate biomarkers TSP2, HA, MMP2, and MMP12 were detected in human cervical secretions. Human cervical secretions obtained from pregnant women at 37 weeks of gestation were used to determine if the exemplary biomarkers TSP2, MMP2, MMP12, and HA can be specifically detected in cervical secretions. For the detection of TSP2, MMP2, MMP12 the cervical secretions (20% of total specimen) were mixed with electrophoresis loading buffer, and the samples were electrophoresed on a 4% (for TSP2 and MMP2), and a 10% (for MMP12) polyacrylamide gel. Western blotting and the washing of the membranes were performed as previously described (52). TSP2 was detected using a monoclonal antibody (BD Biosciences) at 1:100 dilution; MMP2 was detected using a monoclonal antibody (Calbiochem) at 1:100 dilution; MMP12 was detected using a polyclonal antibody (Santa Cruz Biotechnology) at 1: 100 dilution. The horseradish peroxidase-conjugated secondary antibodies were used at 1:500 dilution. Reactive bands were developed using diaminobenzidine substrate.

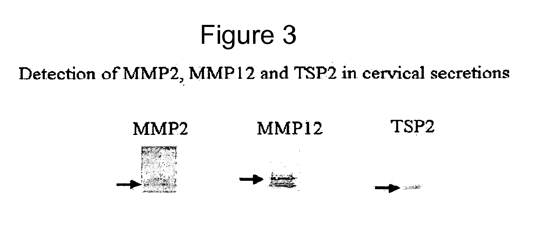

The specific reactive bands are marked by arrows (Figure 3). The molecular weight of MMP2 and MMP12 bands were according to expectations (72 kDa, and 54 kDa). The MMP 12 Western shows additional, lower molecular weight bands, a possible sign of proteolytic processing of the pro-enzyme. The molecular weight of the TSP2 band was smaller than the expected 62 kDa, which indicates proteolytic degradation of TSP2 in cervical secretion.

HA from cervical secretions was detected using the spot-ELISA method. The cervical secretion (10% of the total specimen) was adjusted to contain 1 M guanidine HCl and 5% mercaptoethanol, to disrupt mucus aggregates. The mixture was centrifuged at 15,000 x g for 5 minutes, and the supernatants were loaded onto PVDF membrane using a dot-blotting apparatus. The membranes were processed in an identical manner to the Western blots with the exception that a biotinylated HA-binding protein was substituted for the primary antibody, and a peroxidase-conjugated avidin compound was substituted for the secondary antibody.

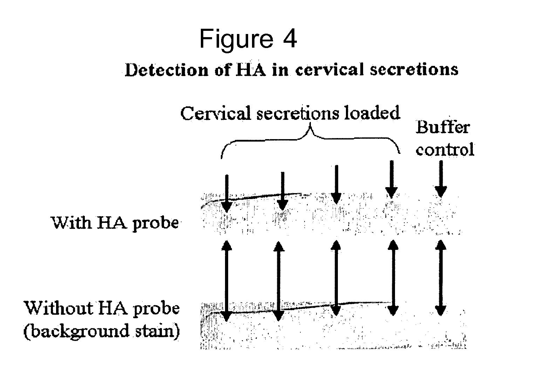

The application of the HA probe resulted in dark circles (top row of dots marked by arrows in Figure 4) indicate detection of HA. The buffer control dot, and the dots where the HA probe was omitted, gave no visible staining signal. These data suggest that HA is specifically detectable in cervical secretions.

### Example 6: Cervical Secretion Sample Collection and Analysis

Sample collection: Cervical secretions are collected from patients with Dacron swabs. The exposed ectocervical surface and the posterior fornix are swabbed, and the swabs are placed into a collecting vial containing physiological saline, Trizma buffer, protease inhibitors (EDTA, phenylmethylsulfonyl fluoride, pepstatin), and antibacterial agent (sodium azide). This sample collection process is very similar to that used for other routine clinical assays (fetal fibronectin) that use cervical secretions as the starting material (See Coleman et al., "Fetal fibronectin detection in preterm labor: evaluation of a prototype bedside dipstick technique and cervical assessment," Am J Obstet Gynecol. 1998 Dec;179(6 Pt 1): 1553-8, which is incorporated herein by reference.

Sample preparation for analysis: The biomarkers are solubilized before loading the specimens onto membranes. A concentrated urea/DTT solution is added to the collecting vial, to bring the final concentrations to 1 M urea, and 10 mM DTT (dithiothreitol) and the swabs are agitated in order to achieve complete release of the macromolecules into the solution. The swabs are removed and the resultant sample is split into aliquots. Sodium dodecyl sulfate (SDS) is added to a final concentration of 1 % to a subset of aliquots, which are then heated to boil, centrifuged at 15,000 x g for 10 minutes. The supernatants are used for the detection of TSP2, MMP2 and MMP12, and for other protein quantification. Another aliquot (or subset of aliquots) is (are) centrifuged at 15,000 x g for 10 minutes, and the supernatant is used for the detection of HA or other glycosaminoaglycans. These biomarkers are water soluble in buffer, and are expected to be in the supernatant.

Spot-ELISA analysis with color development: The clarified samples are loaded into a dot-blotting apparatus, and the solution is drawn through a high-affinity membrane (nitrocellulose membrane for protein biomarkers [e.g., TSP2, MMP12, MMP2], and PVDF membrane for non-protein biomarkers [e.g., HA]). Nitrocellulose membrane is then treated with 5% dry milk solution (in Tris-buffered saline) for 30 minutes. Then the membranes are incubated with the primary antibodies specific for protein biomarkers, e.g., TSP2, MMP2 and MMP12. PVDF membrane is treated with 0.2% BSA (bovine serum albumin) solution (in Tris-buffered saline) for 30 minutes. Then, in the case of HA as the biomarker, the membrane is incubated with a biotinylated hyaluronan-binding protein (purchased from Calbiochem). The incubation proceeds for approximately 12 hours at 4°C in the initial tests. After the incubation the membranes are washed with Tris-buffered saline (containing 0.3% Tween 20), and then incubated with horseradish peroxidase-conjugated secondary antibodies (for TSP2, MMP2 and MMP12), or horseradish peroxidase-conjugated avidin (for HA). After this incubation the membranes are washed several times with Tris-buffered saline (containing 0.3% Tween 20), and the color development performed using diaminiobenzidine substrate.

Chemiluminescence-based detection: In an alternative embodiment to the colorimetric read-out (supra), the invention can be performed using chemiluminescence -based readout of the assays. This chemiluminescence -based assay utilizes the general Spot-Elisa platform described above; however, during the development of signal a chemiluminescence-generating substrate (such as Luminol) is used instead of the diaminiobenzidine substrate. The detection of the chemiluminescence signal and the densitometric quantification may be carried out using an imaging system, such as the Kodak Gel Logic 440 Imaging Station, which is capable of recording chemiluminescence signal with the sensitivity of photographic film. This instrument includes an image analysis software to perform quantitative analysis of the detected signals.

Internal Standards: Given the relative nature of the instant biomarker-based tool, one or more internal standards are used to establish a base-line and to control for varying amounts of collected samples. Internal standards include those proteins that are found in cervical secretions in uniform amounts regardless of the state of the cervix during the progression of pregnancy.

Standard curves for spot-ELISA: Known amounts of recombinant or purified MMP2, MMP12, and HA are spotted in increasing quantities on the membranes. These materials are commercially available from Calbiochem/EMD Biosciences (La Jolla, CA). At present, there is no commercially available purified TSP2, so RSV-3T3 cell lysate from BD Biosciences (San Diego, CA) are used as a uniform source of TSP2. These known materials are dissolved in the buffer used for loading the dot blots. The detection method for the standards is identical to that described (supra). Standard curves are derived by plotting the optical density determined by densitometry against the amount of biomarker.

Densitometry: Optical densities of the stained spots on the spot-ELISA assays are determined by analyzing the digitized images of the ELISA membranes, and analyzing the optical density using the embedded image analysis software in the Kodak Image Station. The standard curves for HA, MMP2 and MMP12 allow for the determination of the absolute amount of biomarkers in the samples. The standard curve for TSP2 will allow for the determination of TSP2 amounts as arbitrary units of optical density. The amounts of biomarkers are then normalized to unit protein level.

Protein quantitation: Optical densities of the spot-ELISAs are normalized to protein levels in the cervical secretions. Protein content of the samples will be determined by the method of Chapdelaine et al (46). This method is based on using amido black solution on membrane-bound proteins, and achieving quantitation of the color intensity by densitometry. This method has been successfully used to quantitate protein levels in cell lysates (47).

### Example 7: Dot blotting protocol for testing of cervical secretions

Cervical secretions were collected from vaginal fornix using Dacron swab. Each swab of cervical secretions was placed into 15 ml blue-top tubes containing 1.5 ml of ice-cold sample collection buffer (10 mM Tris/HCl, pH: 7.4, 0.15 M NaCl, 20 mM EDTA, 5 uM pestatin A, 1 uM leupeptin, 10 ug/ml aprotinin). The samples may be stored in a refrigerator for several days before placing them into -20°C freezer for longer term storage.

For extraction the samples were thawed and 1.5 ml of cold extraction buffer (10 mM Tris/HCl, pH: 7.4, 2 M urea, 0.3 M NaCl, 20 mM EDTA) was added to the tubes. The tubes (still containing the swabs) were vortexed for 30 seconds, and then the swabs were removed and discarded. The extracts were aliquoted into two 1.5 ml microcentrifuge tubes for storage in -20°C freezer.

For analysis the frozen extracts were thawed on ice, and centrifuged in a microcentrifuge at top speed for 10 minutes. For protein quantification, 2 ml of the supernatants were used, for immuno-dot blotting 100 ml of the supernatants were used. Protein quantitation was done using the amido-black staining method of Chapdelaine et al., Biotechiques, 2001, 31/478-482 (which is herein incorporated by reference). The BSA standards were dissolved in 0.5 X extraction buffer.

Immuno-dot blotting was carried out using PVDF (Immobilon-P) membranes. The extracts (100 ml) were drawn through the membrane with the aid of a vacuum pump. The membranes were briefly rinsed with tris-buffered saline (TBS; see Sambrook, Fritsh and Maniatis, "Molecular Cloning: A Laboratory Manual, 2^{nd} Ed.," CSHLP 1989, CSH, New York, which is herein incorporated by reference) and then incubated with 5% dry milk in TBS for 1 hour at room temperature.

The primary antibodies were diluted in dry-milk/TBS at 1:100 dilution from the commercially-available antibody solutions. The antibodies were incubated with the membranes for 14 hours at 4 °C. The membranes were then washed 3 times for 10 minutes each with TBS containing 0.3% (v,v) Tween-20. The membranes were then incubated with horseradish peroxidase-conjugated secondary antibodies for 1 hour at room temperature. The secondary antibodies were diluted in dry-milk/TBS at 1:500 dilution from the commercially-available antibody solutions. The membranes were then washed 3 times 10 minutes each with TBS containing 0.3% Tween-20.

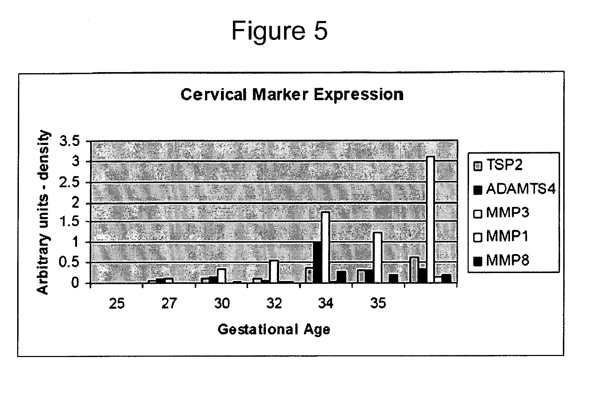

The membranes were developed by incubating them with a solution containing Tris/HCL, pH 7.4, 0.6 mg/ml diaminobenzidine, 0.03% NiCl, and 0.003% hydrogen peroxide. The developed blots were dried, and photographed using a digital camera. Densitometric analyses of the protein blots and the immuno-dot blots were done using the ImageJ (a public domain Java image processing program available freely at http://rsb.info.nih.gov/ij/docs/intro.html.) Densitometry results are presented in Figure 5, which clearly depicts a change in matrix protein expression as pregnancy advances.

### Example 8: Prediction of Preterm Labor

In a clinical trial of human pregnant patients, the candidate biomarkers TSP2, decorin, and MMP12 were shown to have increased levels in cervical secretions of pregnant women destined to enter preterm labor.

56 pregnant women were enrolled into a clinical study. All women were asymptomatic primigravidas (showed no signs of preterm labor). The women consented to provide a specimen of cervical secretions that were collected on a cervical swab. The cervical secretions were extracted with a urea solution, and the extracts were studied in a dot-blotting format. Triplicate aliquots of each specimen were applied to the membrane. Protein levels were determined using the Amido Black technique (58). Levels of TSP2, decorin, and MMP12 were determined using specific antibodies utilizing a chemiluminescent detection method. The chemiluminescent signal was captured on x-ray film, and the dark spots on the film were analyzed by densitometry to obtain optical density values. The darker spot gives rise to higher optical density which indicates stronger chemiluminescence signal, and therefore, more biomarker. The optical density values were normalized to protein content of the specimens to provide the final measure of optical density unit/microgram protein.

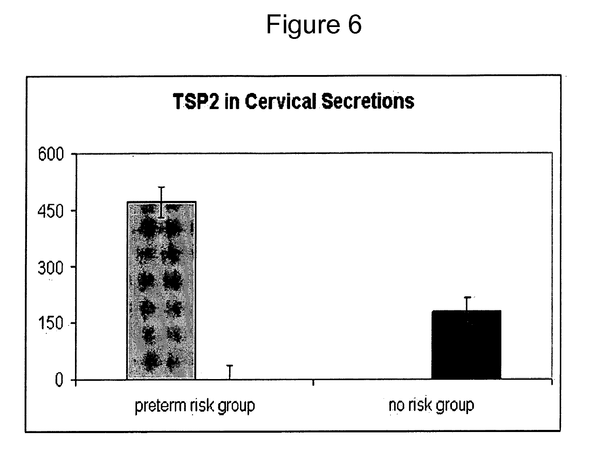

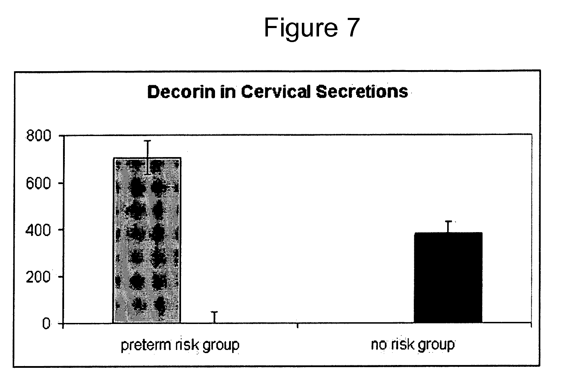

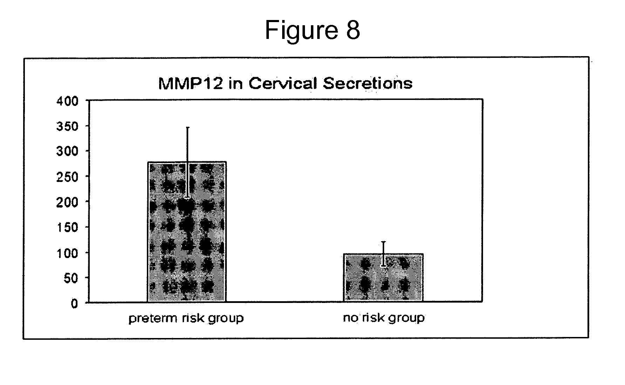

In the patient population ,three women were found who were asymptomatic at the time of specimen collection (at gestational ages of 27 and 30 weeks), and then they were diagnosed with symptoms of preterm labor at 33 weeks gestation (Preterm Risk Group on Figures 6, 7, and 8).

Within the 27-30 week gestational age range, there was a matching group of 10 women whose cervical secretions were collected at 27-30 weeks gestation, but these women were never showed signs of preterm labor (No Risk Group on Figures 6, 7 and 8).

The means of the Preterm Risk group and the No Risk Group were compared using a t test for unequal sample sizes and unequal variances. TSP2 and the decorin levels (expressed in optical density/microgram protein units) were significantly higher (p< 0.01) in women in the Preterm Risk Group (n=3), when compared to women in the No Risk Group (n=10). This preliminary data suggest that these three cervical biomarkers should be investigated in a larger number of study subjects.

## Claims

1. A method for determining the risk of a pregnant patient going into preterm labor comprising:
a. detecting the presence of a biomechanical associated marker in a cervical secretion sample obtained from the patient, wherein the biomechanical associated marker is selected from the group consisting of thrombospondin 2 and decorin and combinations thereof; and
b. correlating an increased level of the biomechanical associated marker, as compared to a normalized baseline marker level, to an increased risk of preterm labor.

2. The method of claim 1 wherein the biomechanical associated marker in the cervical secretion sample comprises thrombospondin 2.

3. The method of any one of claims 1-2 wherein the biomechanical associated marker in the cervical secretion sample comprises decorin.

4. The method of any one of claims 1-3, wherein the biomechanical associated marker in the cervical secretion sample comprises thrombospondin 2 and decorin

5. The method of any one of claims 1-4, further comprising detecting the presence of matrix metalloproteinase-12 and correlating an increased level of matrix metalloproteinase-12 , as compared to a normalized baseline marker level, to an increased risk of preterm labor.

6. The method of any one of claims 1-5 further comprising the determination of an appropriate treatment protocol for the patient,,wherein the appropriate treatment protocol for the patient includes at least one of the following treatments,
i) physical relocation of patient,
ii) administration of drug therapy to patient,
iii) recommendation of a behavioral change to patient, and
iv) closer monitoring of patient.

7. The method of any one of claims 1-6 wherein the drug therapy to be administered to the patient is progesterone or derivatives of progesterone.

8. The method of any one of claims 1-6 wherein the drug therapy to be administered to the patient is a compound that stimulates or blocks uterine contractions.

9. The method of claim 8 wherein the compound that stimulates uterine contractions is pitocin, or oxytocin.

10. The method of claim 8 wherein the compound that inhibits uterine contractions is an oxytocin analogue.

11. The method of any one of claims 1-5 which is performed before, during, or after administration of drug therapy.

12. The method of any one of claims 1 to 11, wherein detecting the presence of a biomechanical associated marker is carried out by measuring the amount of nucleic acid molecules coding for the biomechanical associated marker in the cervical secretion sample.

13. The method of claim 12 wherein the nucleic acid molecule is RNA or DNA.

14. The method of any one of claim 13 wherein the DNA is a cDNA.

15. A kit suitable for the use in determining the risk of a patient going into preterm labor comprising:
a. a swab;
b. collection buffer;
c. an assay for the detection of an increased level of a biomechanical associated marker selected from the group consisting of thrombospondin 2 and decorin and combinations thereof in a cervical secretion sample; packaged in a suitable sealed container.

16. A kit according to claim 15 wherein the assay for the detection of an increased level of a biomechanical associated marker is further capable of detecting levels of matrix metalloproteinase-12, matrix metalloproteinase-8, matrix metalloproteinase-1, matrix metalloproteinase-2, matrix metalloproteinase-3, matrix metalloproteinase-9, hyaluronidase, lumican, fibromodulin, keratocan, PRELP biglcan, mimecan, serglycin, perlecan, agrin, versican, link proteins, CD44, TSG-6, bikunin, inter alpha trypsin inhibitor, a hyaluranan synthase, hyaluronic acid, elastin, fibulins, fibrillins, tenascin X, heparan sulfate, keratan sulfate, chondroitin sulfate, dermatan sulfate, heparanase, lysyl oxidase, type V collagen, type VI collagen, type XII collagen, type XIV collagen, type XVIII collagen, type XX collagen, calreticulin, CD47, syndecans, glypicans, lipoprotein receptor-like protein (LRP), an ADAM protease, an ADAMTS protease, E-selectin, L-selectin, platelet factor 4, transforming growth factor beta 1, 2 and 3 (TGF-β), epidermal growth factor (EGF), keratinocyte growth factor (KGF), vascular endothelial growth factor (VEGF), hepatocyte growth factor (HGF), fibroblast growth factors (FGF) 1 and 2, and platelet derived growth factor (PDGF), and combinations thereof.

17. A kit according to claim 15 or claim 16 wherein the assay for the detection of an increased level of a biomechanical associated marker in the cervical secretion sample is performed by an assay selected from the group consisting of a radioimmunoassay, an enzyme immunoassay, a ligand assay, an immunoradiometric assay, a fluoroimmunoassay, and an enzyme-linked immunosorbent assay.

18. The kit of any one of claims 15-17 wherein the assay for the detection of an increased level of a biomechanical associated marker, is an assay comprising:
a) a primary antibody to the biomechanical associated marker;
b) a secondary antibody that recognizes the primary antibody, or the biomechanical associated marker,
wherein the secondary antibody is labeled by chemiluminescence, a radioactive label, a fluorescent label or an enzymatic label.

19. The kit of any one of claims 15-18 wherein the detection of an increased level of a biomechanical associated marker is accomplished by measuring the amount of nucleic acid molecules coding for a biomechanical associated marker in the cervical secretion sample.

20. The kit of any one of claims 15-19 wherein the nucleic acid molecule to be measured is DNA or RNA.
